# EUROPÄISCHE PATENTANMELDUNG

(11) **EP 1 875 879 A1**
(43) Veröffentlichungstag der Anmeldung: **09.01.2008**
(21) Anmeldenummer: 07111101.7
(22) Anmeldetag: 27.06.2007
(51) Int. Cl.: A61C 19/00, A61L 2/18, A61L 2/22

(54) **Verfahren zur Reinigung und Desinfektion der Kanäle von Dentalinstrumenten**

(30) Priorität: 28.06.2006 EP 06116235; 13.10.2006 EP 06122236
(71) Anmelder: Favodent Karl Huber GmbH, 76229 Karlsruhe (DE)
(72) Erfinder: Wolf, Angela, 76149, Karlsruhe (DE)
(74) Vertreter: Schreiber, Christoph

(57) **Zusammenfassung**

Verfahren zur Reinigung und Desinfektion von Spraykanälen von Dentalinstrumenten umfassend folgende Schritte:
a) Verbinden der Spraykanäle des Dentalinstrumentes mit einer ein Desinfektionsmittel und ein Treibmittel enthaltenden Sprühvorrichtung,
b) Durchspülen der Spraykanäle des Dentalinstrumentes mit dem Desinfektionsmittel aus der Sprühvorrichtung,
c) Einwirken lassen des Desinfektionsmittels.

## Beschreibung

Die vorliegende Erfindung betrifft ein Verfahren zur Reinigung und Desinfektion der Kanäle von Dentalinstrumenten.

In der zahnärztlichen Praxis werden verschiedene Instrumente eingesetzt, die mit Wasser versorgt werden, das aus den dafür vorgesehenen Öffnungen an den Instrumenten austritt. Darüber hinaus weisen die Geräte Kanäle für Sprayluft auf.

Typische Instrumente sind Hand- und Winkelstücke oder Turbinen, wie sie beispielsweise in der DE 196 17 670 A1 beschrieben sind.

Das Wasser hat hierbei mehrere Aufgaben. Es dient zum Kühlen der verschiedenen, in die Instrumente eingesetzten Arbeitswerkzeuge, um ein Überhitzen der Zahnsubstanz infolge von Reibungswärme zu vermeiden. Es hat aber zusätzlich auch die Aufgabe, das von den Zähnen abgetragene Material wegzuspülen, um den Arbeitsbereich zu säubern.

Typischerweise werden die Hand- und Winkelstücke nach dem Einsatz gereinigt, in dem die Außenflächen mit einem alkoholhaltigen Mittel gereinigt und desinfiziert wird.

Das Reinigen und Desinfizieren der in den Instrumenten vorhandenen Innenkanäle (Spraykanäle) ist schwieriger. Das übliche Verfahren ist ein maschinelles Verfahren zur Thermodesinfektion in einem Reinigungs- und Desinfektionsgerät (RDG) (ähnlich einer Geschirrspülmaschine). Hierbei muss auf die richtige Beladung, die Vermeidung von Spülschatten, korrekte Temperatur und Zeit und den Einsatz der richtigen Reinigungsmittel geachtet werden. Die Reinigungs- und Desinfektionsgeräte (RDG) müssen über eine Adaption der Kanäle verfügen. Es existiert allerdings keine Überwachung der Kanäle in den RDGs, ob die Durchspülung während des Reinigungsvorgangs funktioniert. Entsprechende Reinigungs- und Desinfektionsgeräte sind teuer.

EP 0 099 209 A1 beschreibt eine Desinfektionslösung. Die Verwendung von Stickstoff als Treibmittel ist nicht beschrieben, ebenso nicht die Anwendung in Dentalinstrumenten.

WO 02/065838 A1 beschreibt eine Desinfektionslösung. Die Verwendung von Stickstoff als Treibmittel ist nicht beschrieben, ebenso nicht die Anwendung in Dentalinstrumenten.

EP 0 732 083 A2 beschreibt eine Vorrichtung zum Reinigen eines Antriebskanals in einem medizinischen Handstück. Eine Desinfektion der Kanäle ist nicht beschrieben.

Die Aufgabe der vorliegenden Erfindung war es, zumindest einige der genannten Nachteile des Standes der Technik zu überwinden, insbesondere ein Verfahren bereitzustellen, mit dem in einfacher und kostengünstiger Weise eine Desinfektion der Spraykanäle möglich ist.

Gelöst wird die Aufgabe durch ein Verfahren zur Reinigung und Desinfektion von Spraykanälen von Dentalinstrumenten, welches folgende Schritte umfasst:
a) Verbinden der Spraykanäle des Dentalinstrumentes mit einer ein Desinfektionsmittel und ein Treibmittel enthaltenden Sprühvorrichtung,
b) Durchspülen der Spraykanäle des Dentalinstrumentes mit dem Desinfektionsmittel aus der Sprühvorrichtung,
c) Einwirken lassen des Desinfektionsmittels.

Der Begriff "Spraykanäle" umfasst Wasserkanäle und Luftkanäle.

Erfindungsgemäß kann also die Reinigung der Kanäle mittels Durchspülen mit einem Desinfektionsmittel unter Druck erfolgen, so dass auf den Einsatz teurer Geräte wie eines Reinigungs- und Desinfektionsgerätes verzichtet werden kann.

Hierzu wird eine Sprühvorrichtung, die ein Desinfektionsmittel und ein Treibmittel enthält, mit den Wasserkanälen des Dentalinstrumentes verbunden. Es sind gegebenenfalls Adapter vorgesehen, die eine entsprechende Verbindung zwischen der Sprühvorrichtung und den zu reinigenden Instrumenten ermöglichen. Anschließend erfolgt ein Durchspülen der Kanäle mit dem Desinfektionsmittel.

Das in der Sprühvorrichtung vorhandene Treibmittel treibt hierbei das Desinfektionsmittel aus der Dose durch die Kanäle. Das austretende Desinfektionsmittel wird aufgefangen und entsorgt. Bevorzugt handelt es sich bei der Sprühvorrichtung um eine einfache Sprühdose zur Einmalverwendung. Diese weist einen Anschlussstutzen zum Verbinden mit dem Dentalinstrument auf.

Typischerweise genügt ein Durchspülen für eine Zeit zwischen 1 und 20 s, bevorzugt zwischen 1 und 5 oder 5 und 15 s, oder 6 und 12s. Die genaue Zeit ist abhängig von dem Druck der Sprühvorrichtung und der Volumina der Kanäle.

Anschließend muss das Desinfektionsmittel über einen gewissen Zeitraum einwirken. Diese Einwirkzeit liegt vorzugsweise zwischen 30 s und 15 min, bevorzugte Bereiche liegen zwischen 1 und 3 oder 3 und 10 min oder 4 und 8 min. Vor dem Einsatz werden die Spraykanäle vom Desinfektionsmittel befreit, dies erfolgt beispielsweise einfach durch Durchspülen mit Wasser in Trinkwasserqualität oder durch den Einsatz von Druckluft.

Als Desinfektionsmittel sind z.B. Desinfektionsmittel auf Alkoholbasis oder auf Basis quaternärer Ammoniumverbindungen geeignet.

Als besonders geeignet zur Durchführung des Verfahrens hat sich ein Desinfektionsmittel erwiesen, das folgende Zusammensetzung aufweist:
a) 35 bis 50 Gew.-% Ethanol,
b) 5 bis 15 Gew.-% 1-Propanol,
c) 0,03 bis 0,2 Gew.-% einer quaternären Ammoniumverbindung, wie Didecyldimethylammoniumchlorid.

Die Leistungsfähigkeit der Desinfektion scheint auch vom Einsatz des Treibmittels in der Sprühvorrichtung abhängig zu sein. So hat sich Stickstoff als Treibmittel als besonders geeignet erwiesen, da es nicht dazu neigt, aus dem Desinfektionsmittel auszuperlen und dadurch die Benetzung der Fläche zu behindern.

Gegenstand der Vorrichtung ist weiterhin eine Sprühvorrichtung mit einem Desinfektionsmittel und Stickstoff als Treibmittel, sowie die Verwendung der entsprechenden Vorrichtung zur Desinfektion der Spraykanäle von Dentalinstrumenten, insbesondere Hand- und Winkelstücken und Turbinen.

Es handelt sich um ein validiertes Verfahren mit einer Desinfektionsleistung von mehr als 5 log-Einheiten.

Die vorliegende Erfindung wird durch die nachfolgenden Beispiele näher erläutert.

### Beispiel 1

### Herstellung des Desinfektionsmittels

Ein Desinfektionsmittel wurde hergestellt durch Vermischen von 43 Gew.-% Ethanol, 8 Gew.-% 1-Propanol, 0,08 Gew.-% Didecyldimethylammoniumchlorid und 48,92 Gew.-% Wasser. Die ausreichende Desinfektionsleistung einer solchen Zusammensetzung zur Flächendesinfektion ist bekannt. Eine entsprechende Desinfektionslösung wurde in eine Sprühflasche unter Einsatz des Treibmittels Stickstoff abgefüllt.

### Beispiel 2

### Vorbereitung der Übertragungsinstrumente (Turbinen, Hand- und Winkelstücke)

Übertragungsinstrumente sind vom Robert-Koch-Institut (RKI) und dem Bundesinstitut für Arzneimittel und Medizinprodukte (BfArM) in die Risikogruppe Semikritisch B und Kritisch B eingeordnet.

Die Risikogruppe Kritisch B betrifft Medizinprodukte, die die Haut oder Schleimhaut durchdringen oder mit Blut in Kontakt kommen. Dies betrifft rotierende oder oszillierende Instrumente für chirurgische, parodontologische oder endodontische Maßnahmen.

Die Risikogruppe Semikritisch B betrifft Medizinprodukte, die mit Schleimhaut oder krankhaft veränderter Haut in Kontakt kommen. Hierunter fallen rotierende und oszillierende Instrumente für allgemeine, präventive, restaurative oder kieferorthopädische Behandlungen.

Aufgrund des komplexen Aufbaus bestehen bei Übertragungsinstrumenten erhöhte Anforderungen an die Aufbereitung. Wegen der schlecht zugänglichen und daher schlecht bespülbaren Oberflächen entsprechender Geräte ist der Reinigungserfolg nicht visuell überprüfbar.

Entsprechende Hand- und Winkelstücke wurden mit dem Testkeim *Enterococcus hirae* kontaminiert und die mikrobiologische Wirksamkeit in Anlehnung an die DIN EN 1276:1997-08 getestet.

Zum Test wurde ein Winkelstück der Firma Kavo Typ INTRAmatic LUX 2 25 LN eingesetzt, dass vor der Überprüfung durch ein thermisches Reinigungs- und Desinfektionsgerät gereinigt und thermisch desinfiziert.

### Beispiel 3

### Bestimmung der Wiederfindungsrate

Es wurde eine Keimsuspension mit *Enterococcus hirae* auf einen Titer von 3,0 x 10⁹KBE/ml eingestellt. Von dieser Lösung wurden 10 µl in das Innere der Wände des Winkelstücks gespritzt und anschließend 1 h bei Raumtemperatur getrocknet.

Dann wurden 10 ml Nährbouillon in die Kanäle eingespritzt und in einem sterilen Behälter aufgefangen. Die Nährbouillon wurde wieder mit einer Spritze aufgenommen und nachfolgend noch weitere vier Mal durch die Kanäle gespült.

Anschließend erfolgte eine Keimzahlbestimmung. Dies wird als Wiederfindungsrate bezeichnet. Tabelle 1 zeigt die gefundenen Werte

| Versuch | Ausgangskeimzahl [log] | Endkeimzahl [log] | Verlustrate [log] |
|---|---|---|---|
| 1 | 7,48 | 6,61 | 0,87 |
| 2 | 7,48 | 6,46 | 1,02 |
| 3 | 6,69 | 5,99 | 0,70 |
| 4 | 6,69 | 5,88 | 0,81 |
| 5 | 7,84 | 6,87 | 0,97 |
| Mittelwert | | | 0,87 |

### Beispiel 4

### Bestimmung der Keimreduktion durch das erfindungsgemäße Verfahren

Es wurde eine Keimsuspension von *Enterococcus hirae* eingesetzt. Von dieser Suspension wurden 10 µl in die Wasserkanäle des HWS eingespritzt. Hierbei wurde vom Kopf die Suspension als Tropfen sichtbar. Das HWS wurde 1 h bei Raumtemperatur getrocknet. Nach der Trocknung wurde das HWS mittels der erfindungsgemäßen Sprühvorrichtung mit der Desinfektionslösung 2 s gespült.

Nach einer Einwirkzeit von 1 min erfolgte die mikrobiologische Probennahme. Hierzu wurden mittels eines Adapters 10 ml Nährbouillon in die Kanäle eingespritzt und in einem sterilen Behälter aufgefangen. Die Nährbouillon wurde wieder mit der Spritze aufgenommen und nachfolgend weitere 4 Mal durch das HWS gespült.

Im Anschluss daran erfolgte die Keimzahlbestimmung.

Es wurden 10 Versuche durchgeführt, wobei die Versuche an 5 Tagen durchgeführt wurden. An jedem Tag wurden mit einer Bakteriensuspension jeweils 2 Kontaminationsversuche durchgeführt, so dass am Ende 5 verschiedene Bakteriensuspensionen mit entsprechenden Ausgangskeimzahlen zum Einsatz kamen.

**Tabelle 2 zeigt die Bestimmung der Keimreduktion.**

| Versuch | Ausgangskeimzahl [log] | Ausgangskeimzahl nach Korrektur [log] | Keimzahl nach Behandlung [log] | Reduktion [log] |
|---|---|---|---|---|
| 1 | 7,18 | 6,31 | 0 | 6,31 |
| 2 | 7,18 | 6,31 | 0 | 6,31 |
| 3 | 7,34 | 6,47 | 0 | 6,47 |
| 4 | 7,34 | 6,47 | 0 | 6,47 |
| 5 | 6,98 | 6,11 | 0 | 6,11 |
| 6 | 6,98 | 6,11 | 0 | 6,11 |
| 7 | 7,02 | 6,15 | 0 | 6,15 |
| 8 | 7,02 | 6,15 | 0 | 6,15 |
| 9 | 7,21 | 6,34 | 0 | 6,34 |
| 10 | 7,21 | 6,34 | 0 | 6,34 |

Es zeigt sich, dass bei allen durchgeführten Versuchen keine Keime mehr nachgewiesen werden konnten. Die Keimzahlreduktion ist größer als 5 log-Stufen, so dass ein geeignetes Verfahren zur manuell-chemischen Desinfektion von Wasserkanälen von Dentalinstrumenten vorliegt.

### Beispiel 5

### Prüfung der Reinigungsleistung des erfindungsgemäßen Verfahrens

In einem weiteren Experiment wurde untersucht, ob der Einsatz des erfindungsgemäßen Verfahrens neben der Desinfektion auch eine zufriedenstellende Reinigung erlaubt. Hierzu wurde als Testlösung eine Mischung von heparinisiertem Schafblut, ⁹⁹Technetium markiertem Makroalbuminaggregaten (Pulmocis^{®}), Protaminsulfat und Pufferlösung hergestellt. Die dentalen Winkelstücke wurden mit der Testverschmutzung verunreinigt und für 10 min zum Antrocknen belassen. Danach wurden die Handstücke hinsichtlich der Counts an Technetium vermessen. Anschließend wurden die Handstücke zunächst mit entionisiertem Wasser durchgespült und dann mittels des erfindungsgemäßen Verfahrens behandelt. Nach einer einminütigen Einwirkzeit wird erneut mit Wasser durchgespült. Es zeigte sich, dass nur ca. 20% des Technetium wiedergefunden wurde. Dies entspricht Werten, wie sie auch mit den aufwändigeren Reinigungs- und Desinfektionsgeräten (RDG) gefunden werden.

## Patentansprüche

1. Verfahren zur Reinigung und Desinfektion von Spraykanälen von Dentalinstrumenten umfassend folgende Schritte:
a) Verbinden der Spraykanäle des Dentalinstrumentes mit einer ein Desinfektionsmittel und ein Treibmittel enthaltenden Sprühvorrichtung,
b) Durchspülen der Spraykanäle des Dentalinstrumentes mit dem Desinfektionsmittel aus der Sprühvorrichtung,
c) Einwirken lassen des Desinfektionsmittels.

2. Verfahren nach Anspruch 1, **dadurch gekennzeichnet, dass** das Desinfektionsmittel folgende Zusammensetzung aufweist:
a) 35 bis 50 Gew.-% Ethanol,
b) 5 bis 15 Gew.-% 1-Propanol,
c) 0,03 bis 0,2 Gew.-% einer quaternären Ammoniumverbindung.

3. Verfahren nach Anspruch 1 oder 2, **dadurch gekennzeichnet, dass** die Sprühvorrichtung als Treibmittel Stickstoff enthält.

4. Verfahren nach mindestens einem der Ansprüche 1 bis 3, **dadurch gekennzeichnet, dass** die Zeit, in der das Desinfektionsmittel durchgespült wird, zwischen 1 und 20 s beträgt.

5. Verfahren nach mindestens einem der Ansprüche 1 bis 4, **dadurch gekennzeichnet, dass** die Zeit, in der das Desinfektionsmittel einwirkt, zwischen 30 s und 15 min liegt.

6. Verfahren nach mindestens einem der Ansprüche 1 bis 5, **dadurch gekennzeichnet, dass** die Spraykanäle Wasserkanäle und Luftkanäle sind.

7. Sprühvorrichtung mit einem Desinfektionsmittel und Stickstoff als Treibmittel.

8. Sprühvorrichtung nach Anspruch 6, **dadurch gekennzeichnet, dass** das Desinfektionsmittel
a) 35 bis 50 Gew.-% Ethanol,
b) 5 bis 15 Gew.-% 1-Propanol
c) und 0,03 bis 0,2 Gew.-% einer quaternären Ammoniumverbindung, wie Didecyldimethylammoniumchlorid
enthält.

9. Verwendung einer Sprühvorrichtung nach Anspruch 7 oder 8 zur Desinfektion der Spraykanäle von Dentalinstrumenten, insbesondere nach dem Verfahren gemäß Ansprüche 1 bis 6.
